(19) Europäisches Patentamt
European Patent Office
Office européen des brevets

(11) **EP 2 944 265 A1**

(12) **EUROPEAN PATENT APPLICATION**
published in accordance with Art. 153(4) EPC

(43) Date of publication:
**18.11.2015 Bulletin 2015/47**

(51) Int Cl.:
***A61B 8/00*** *(2006.01)*

(21) Application number: **13871001.7**

(86) International application number:
**PCT/JP2013/084685**

(22) Date of filing: **25.12.2013**

(87) International publication number:
**WO 2014/109234 (17.07.2014 Gazette 2014/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.01.2013  JP 2013001824**

(71) Applicant: **Hitachi Aloka Medical, Ltd.**
**Tokyo 181-8622 (JP)**

(72) Inventor: **HISATSU, Masanori**
**Mitaka-shi**
**Tokyo 181-8622 (JP)**

(74) Representative: **Heim, Florian Andreas et al**
**Weber & Heim**
**Patentanwälte**
**Partnerschaftsgesellschaft mbB**
**Irmgardstraße 3**
**81479 München (DE)**

(54) **ULTRASONIC DIAGNOSTIC DEVICE**

(57)    An evaluation value calculation unit (50) evaluates a degree of phasing on the basis of a plurality of received-wave signals obtained from a delay processing unit (30) to thereby calculate a two-dimensional evaluation value related to a two-dimensional array of a plurality of oscillating elements (12). The evaluation value calculation unit (50) obtains a two-dimensional evaluation value of an xy plane from a one-dimensional evaluation value in the x direction and a one-dimensional evaluation value in the y direction. A multiplication unit (60) multiplies the two-dimensional evaluation value and the received beam outputted from an addition processing unit (40) to adjust the gain of the received beam. Unnecessary signal components are thereby reduced.

FIG. 1

**Description**

Technical Field

The present invention relates to an ultrasound diagnostic apparatus, and in particular to a technique for reducing an unnecessary signal component.

Background Art

In improving the image quality of an ultrasound image, it is desirable to reduce an unnecessary signal component such as a side lobe and a grating lobe included in a received signal. In formation of a reception beam of ultrasound, a plurality of received wave signals obtained from a plurality of transducer elements are delay-processed according to a focus position, and phases of reflection waves from the focus position are matched. In other words, the plurality of received wave signals are phased. The plurality of received wave signals to which the delay process is applied are summation-processed, to form a reception beam signal.

However, the received wave signal obtained from each transducer element also includes unnecessary reflection wave components from positions other than the focus position. Because of this, the reception beam signal obtained by summation-processing the plurality of received wave signals to which the delay process is applied includes an unnecessary signal component due to the unnecessary reflection wave component. Techniques for reducing the unnecessary signal component have been proposed in the related art.

For example, Patent Document 1 discloses a technique to reduce the unnecessary signal component using a CF (Coherence Factor) that indicates the degree of phasing. In addition, as a value indicating the degree of phasing, Non-Patent Document 1 discloses a PCF (Phase Coherence Factor) and an SCF (Sign Coherence Factor), Patent Document 2 discloses an STF (Sign Transit Factor), and Non-Patent Document 2 discloses a GCF (Generalized Coherence Factor).

[Related Art References]

[Patent Documents]

[Patent Document 1] USP 5,910,115
[Patent Document 2] JP 2012-81114 A

[Non-Patent Documents]

[Non-Patent Document 1] J. Camecho, et al., "Phase Coherence Imaging", IEEE trans. UFFC, vol. 56, No. 5, 2009
[Non-Patent Document 2] Pai-Chi Li, et al., "Adaptive Imaging Using the Generalized Coherence Factor", IEEE trans. UFFC, vol. 50, No. 2, 2003

Disclosure of Invention

[Technical Problem]

The values such as CF indicating the degree of phasing are particularly preferable in reducing the unnecessary signal component related to a one-dimensional array transducer in which a plurality of transducer elements are arranged one-dimensionally.

In such a circumstance, the present inventors have conducted research and development for reduction of the unnecessary signal components in a two-dimensional array transducer in which a plurality of transducer elements are arranged two-dimensionally.

The present invention has been conceived in the course of the research and development, and an advantage of the present invention lies in provision of a technique to reduce an unnecessary signal component in a two-dimensional array transducer.

[Solution to Problem]

According to one aspect of the present invention, there is provided an ultrasound diagnostic apparatus comprising: a plurality of transducer elements that are two-dimensionally arranged; a delay processor that applies a delay process on a plurality of received wave signals obtained from the plurality of transducer elements, to phase the received wave signals; an evaluation value calculation unit that evaluates a degree of phasing based on the plurality of received wave signals to which the delay process is applied, to obtain a two-dimensional evaluation value related to the plurality of transducer elements that are two-dimensionally arranged; a summation processor that applies a summation process to the plurality of received wave signals to which the delay process is applied, to obtain a reception signal; and a signal adjustment unit that adjusts the reception signal based on the two-dimensional evaluation value, to reduce an unnecessary signal component. According to another aspect of the present invention, preferably, the evaluation value calculation unit evaluates, for the plurality of transducer elements that are two-dimensionally arranged, a degree of phasing for each arrangement direction for a plurality of arrangement directions that differ from each other, to calculate a one-dimensional evaluation value, and calculates the two-dimensional evaluation value indicating a degree of phasing over the entirety of the two-dimensional arrangement based on a plurality of the one-dimensional evaluation values obtained from the plurality of arrangement directions.

In the above-described apparatus, the plurality of transducer elements are arranged in a lattice shape; for example, along a lateral direction (x direction) and a vertical direction (y direction), and form a two-dimensional array transducer. A transducer plane formed by the plurality of transducer elements may have a rectangular shape or a circular shape. In addition, the adjacent transducer elements may be arranged to be shifted from each other, or some of the transducer elements may be set as ineffective elements, to form a sparse-type two-dimensional array transducer.

The evaluation value calculation unit evaluates the degree of phasing for each arrangement direction of the plurality of transducer elements, to calculate a one-dimensional evaluation value. The degree of phasing refers to a degree related to a state of phases for a plurality of received wave signals to which the delay process is applied, and, for example, the evaluation of the degree of phasing includes evaluation of the degree of match of the phases or the degree of shifting in the phases. Because it is sufficient for the evaluation value calculation unit to calculate the degree of phasing one-dimensionally, as the one-dimensional evaluation value, there may be used, for example, the CF (Coherence Factor) of Patent Document 1, the PCF (Phase Coherence Factor) and the SCF (Sign Coherence Factor) of Non-Patent Document 1, and the STF (Sign Transit Factor) of Patent Document 2, or the like. Alternatively, one-dimensional evaluation values other than these values may be used.

The evaluation value calculation unit calculates a two-dimensional evaluation value indicating the degree of phasing over the entirety of the two-dimensional arrangement based on a plurality of the one-dimensional evaluation values obtained from a plurality of arrangement directions. For example, the two-dimensional evaluation value may be calculated by a relatively simple calculation such as summation, multiplication, square-summation, or the like, of the plurality of one-dimensional evaluation values.

The reception signal obtained by summation-processing the plurality of the received wave signals to which the delay process is applied is adjusted based on the two-dimensional evaluation value. The signal adjusting unit changes, for example, a gain (amplitude) of the reception signal according to the size of the two-dimensional evaluation value. Alternatively, the phase of the reception signal or the like may be adjusted based on the two-dimensional evaluation value as necessary. By the signal adjusting unit adjusting the reception signal based on the two-dimensional evaluation value, the unnecessary signal component is reduced.

According to another aspect of the present invention, preferably, the evaluation value calculation unit calculates, for each arrangement direction, the one-dimensional evaluation value for the arrangement direction based on a plurality of received wave signals obtained from transducer elements of at least one line along the arrangement direction.

According to another aspect of the present invention, preferably, the evaluation value calculation unit calculates the one-dimensional evaluation value for each arrangement direction for two arrangement directions that are orthogonal to each other, and calculates the two-dimensional evaluation value based on two one-dimensional evaluation values obtained from the two arrangement directions.

[Advantageous Effects of Invention]

According to various aspects of the present invention, a technique for reducing an unnecessary signal component in a two-dimensional array transducer is provided. For example, according to a preferred form of the present invention, a two-dimensional evaluation value indicating a degree of phasing over the entirety of a two-dimensional arrangement of a plurality of transducer elements is calculated based on a plurality of one-dimensional evaluation values obtained from a plurality of arrangement directions, and a reception signal is adjusted based on the two-dimensional evaluation value, so that the unnecessary signal component is reduced.

Brief Description of Drawings

FIG. 1 is a diagram showing an overall structure of an ultrasound diagnostic apparatus according to a preferred embodiment of the present invention.
FIG. 2 is a diagram showing a specific example of a plurality of transducer elements 12 that are two-dimensionally arranged.
FIG. 3 is a diagram showing a coordinate system with reference to a two-dimensional array transducer 10.
FIG. 4 is a diagram showing a wave plane of a received wave signal after a delay process.

Embodiment

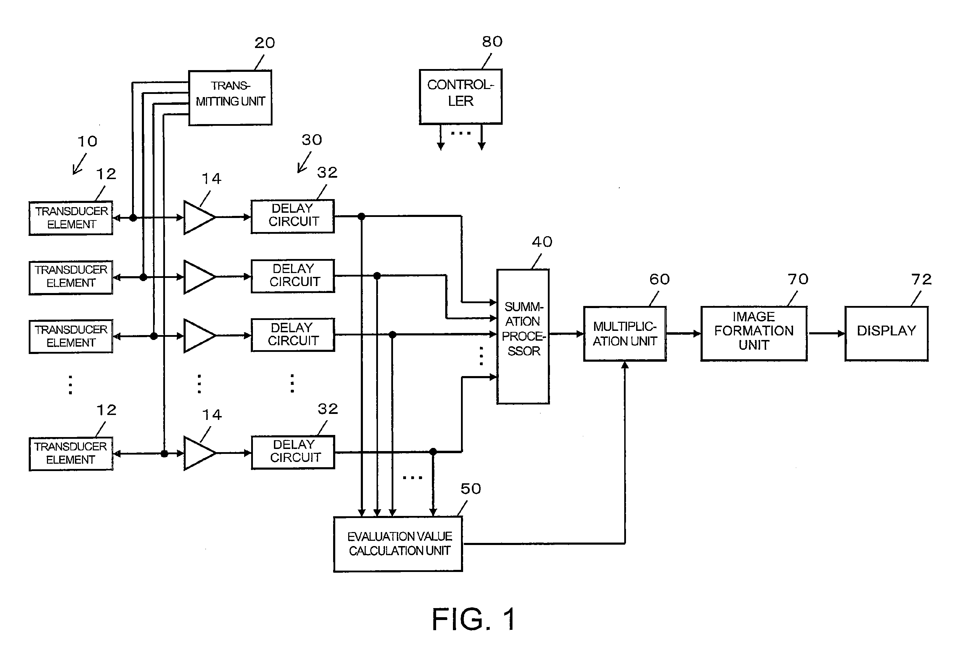

FIG. 1 is a block diagram showing the overall structure of an ultrasound diagnostic apparatus according to a preferred embodiment of the present invention. Each of a plurality of transducer elements 12 is an element that transmits and receives an ultrasound, and the plurality of transducer elements 12 are two-dimensionally arranged to form a two-dimensional array transducer 10. The two-dimensional array transducer 10 is an ultrasound probe for a three-dimensional image that three-dimensionally scans an ultrasound beam in a three-dimensional diagnostic region. The two-dimensional array transducer 10 may three-dimensionally scan the ultrasound beam electrically or by a combination of electrical scanning and mechanical scanning.

A transmitting unit 20 outputs a transmission signal to each of the plurality of transducer elements 12 of the two-dimensional array transducer 10, transmission-controls the plurality of transducer elements 12 to form a transmission beam, and scans the transmission beam in a diagnostic region. In other words, the transmitting unit 20 has a function of a transmission beam former.

Each transducer element 12 is transmission-controlled by the transmitting unit 20 to transmit an ultrasound wave, and receives an ultrasound wave obtained from the diagnostic region in response to the transmitted wave. A received wave signal obtained by each transducer element 12 receiving the ultrasound is sent from each transducer element 12 to a delay processor 30 through a pre-amplifier 14.

The delay processor 30 comprises a plurality of delay circuits 32. Each delay circuit 32 applies a delay process to a received wave signal obtained from the corresponding transducer element 12 through the corresponding pre-amplifier 14. With such a configuration, the plurality of received wave signals obtained from the plurality of transducer elements 12 are delay-processed according to a focus position, and the phases of the reflection waves from the focus position are matched. In other words, the plurality of received wave signals are phased. The plurality of received wave signals to which the delay process is applied are summation-processed at a summation processor 40, to form a reception beam signal.

In this manner, the phased-summation process is executed by the delay processor 30 and the summation processor 40, to realize a function of a reception beam former. A reception signal is scanned over the entirety of the diagnostic region to follow the scanning of the transmission beam, and a reception beam signal is collected along the reception beam.

An evaluation value calculation unit 50 evaluates a degree of phasing based on a plurality of received wave signals obtained from the delay processor 30, to calculate a two-dimensional evaluation value related to the two-dimensional arrangement of the plurality of transducer elements 12. A multiplication unit 60 multiplies the reception beam signal output from the summation processor 40 and the two-dimensional evaluation value, to adjust a gain of the reception beam signal. With this process, the unnecessary signal component is reduced. In other words, the multiplication unit 60 functions as a signal adjusting unit that adjusts the reception signal based on the two-dimensional evaluation value.

An image formation unit 70 forms a three-dimensional ultrasound image based on the reception beam signal adjusted by the multiplication unit 60. The image formation unit 70 forms an ultrasound image three-dimensionally representing a diagnosis target using, for example, a volume rendering method. Alternatively, the image formation unit 70 may form a tomographic image (B-mode image) or a Doppler image of the diagnosis target. The ultrasound image formed by the image formation unit 70 is displayed on a display 72 realized by a liquid crystal display or the like. A controller 80 integrally controls the entirety of the ultrasound diagnostic apparatus of FIG. 1.

Of the structures (functional blocks) shown in FIG. 1, each of the pre-amplifier 14, the transmitting unit 20, the delay processor 30, the summation processor 40, the evaluation value calculation unit 50, the multiplication unit 60, and the image formation unit 70 may be realized using hardware such as, for example, an electronic/electric circuit, a processor, or the like, and devices such as a memory may be used as necessary in realization of these structures. In addition, the controller 80 maybe realized, for example, by cooperation between hardware such as a CPU, a processor, and a memory, and software (program) that defines operations of the CPU and the processor.

The ultrasound diagnostic apparatus of FIG. 1 has been summarized. Next, the two-dimensional evaluation value used in the ultrasound diagnostic apparatus of FIG. 1 will be described in detail. The structures (parts) shown in FIG. 1 are referred to by the reference numerals of FIG. 1 also in the following description.

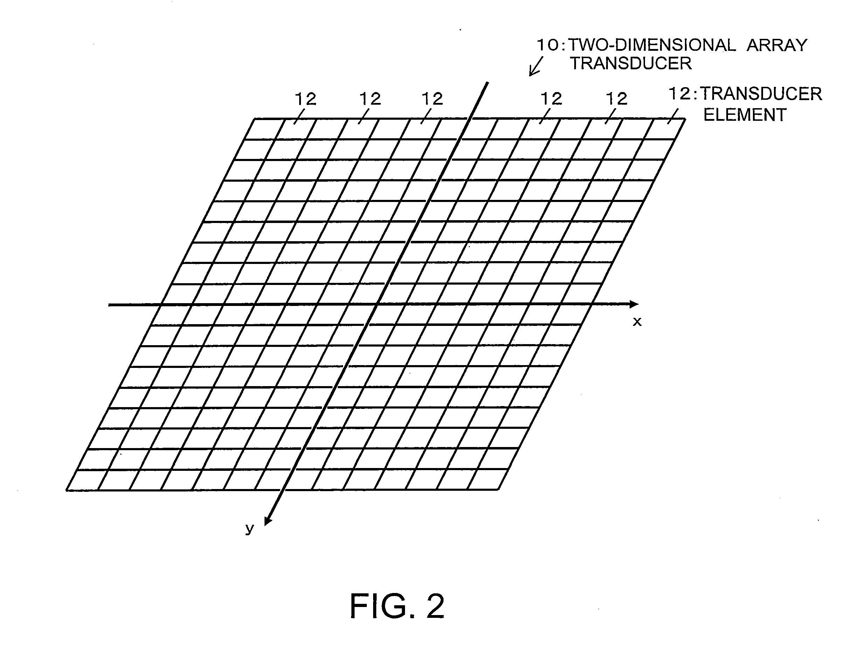

FIG. 2 is a diagram showing a specific example of the plurality of transducer elements 12 that are two-dimensionally arranged. FIG. 2 shows an arrangement state of the plurality of transducer elements 12 of the two-dimensional array transducer 10. In the specific example of FIG. 2, the plurality of transducer elements 12 are arranged along each of an x direction and a y direction orthogonal to each other, and are arranged in a lattice shape, to form a transducer plane of the two-dimensional array transducer 10. The two-dimensional evaluation value is calculated based on the one-dimensional evaluation values for the x direction and the y direction.

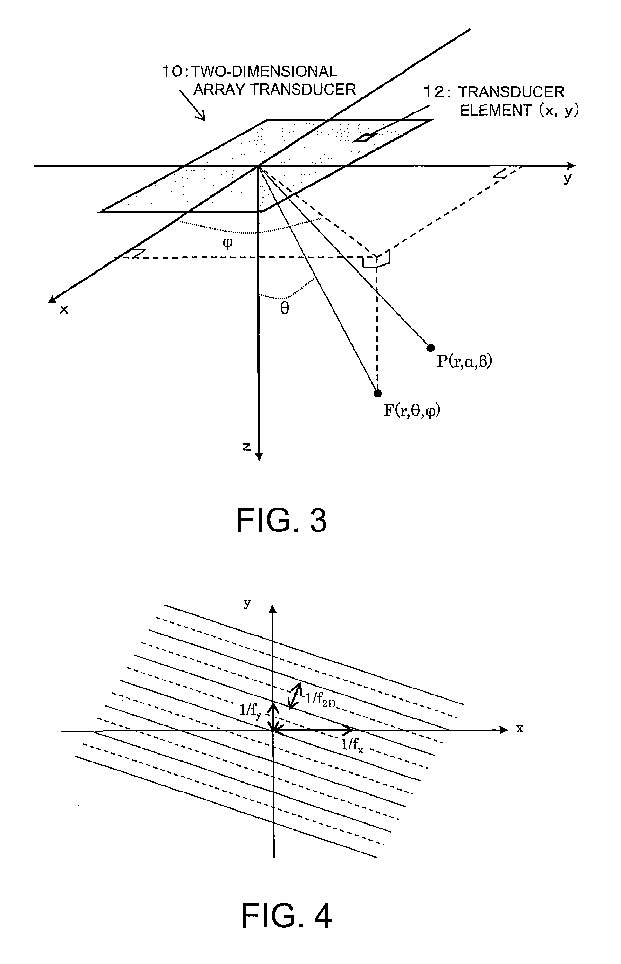

FIG. 3 is a diagram showing a coordinate system with reference to the two-dimensional array transducer 10. FIG. 3 shows an xyz orthogonal coordinate system and an rθφ polar coordinate system having a center of the transducer plane of the two-dimensional array transducer 10 as an origin.

A propagation distance of ultrasound from a reception focus point F (r, θ, φ) to the transducer element 12 (x, y) is calculated by Equation 1. In Equation 1, c represents the speed of ultrasound, and $t_f$ represents the propagation time of the ultrasound.

[Equation 1]

$$c \cdot t_f = \sqrt{\left(r \sin\theta \cos\phi - x\right)^2 + \left(r \sin\theta \sin\phi - y\right)^2 + \left(r \cos\theta\right)^2}$$

When the absolute values of x and y are both sufficiently smaller than r, Equation 1 may be approximated to Equation 2.

[Equation 2]

$$c \cdot t_f \cong r + \frac{x^2 + y^2}{2r} - \sin\theta \cdot \left(x \cos\phi + y \sin\phi\right)$$

The propagation distance of the ultrasound from a point P (r, α, β) having the same distance from the origin as the reception focus point F (r, θ, φ) to the transducer element 12 (x, y) is represented by Equation 3, by a similar derivation as for Equation 2.

[Equation 3]

$$c \cdot t_p \cong r + \frac{x^2 + y^2}{2r} - \sin\alpha \cdot \left(x \cos\beta + y \sin\beta\right)$$

Therefore, a phase difference between a reflection wave (received wave signal) from the reception focus point F (r, θ, φ) and a reflection wave (received wave signal) from the point P (r, α, β) at the transducer element 12 (x, y) is represented by Equation 4, when the frequency of the ultrasound is f.

[Equation 4]

$$\Delta\psi(x,y) = 2\pi f\left(t_p - t_f\right) = \frac{2\pi f}{c}\left\{-\sin\alpha\cdot\left(x\cos\beta + y\sin\beta\right) + \sin\theta\cdot\left(x\cos\phi + y\sin\phi\right)\right\}$$

$$= \frac{2\pi f}{c}\left\{\left(\sin\theta\cos\phi - \sin\alpha\cos\beta\right)x + \left(\sin\theta\sin\phi - \sin\alpha\sin\beta\right)y\right\}$$

In the two-dimensional array transducer 10, when the number of elements of the transducer elements 12 arranged along the x direction and the number of elements of the transducer elements 12 arranged along the y direction are both N, an element spacing between adjacent transducer elements 12 is $\lambda/2$, the element number of the transducer elements arranged along the x direction is m, and the element number of the transducer elements 12 arranged in the y direction is n, Equation 5 can be derived.

[Equation 5]

$$x = \left(m - \frac{N-1}{2}\right)\cdot\frac{\lambda}{2}, \quad y = \left(n - \frac{N-1}{2}\right)\cdot\frac{\lambda}{2}, \quad 0 \le m \le N-1, \quad 0 \le n \le N-1,$$

Equation 5 may be substituted into Equation 4, to obtain Equation 6.

[Equation 6]

$$\Delta\psi(x,y) = \pi\left\{\left(\sin\theta\cos\phi - \sin\alpha\cos\beta\right)\left(m - \frac{N-1}{2}\right) + \left(\sin\theta\sin\phi - \sin\alpha\sin\beta\right)\left(n - \frac{N-1}{2}\right)\right\}$$

Further, Equation 6 may be simplified, so that the phase difference between the reflection wave (received wave signal) from the reception focus point F (r, θ, φ) and the reflection wave (received wave signal) from the point P (r, α, β) at the transducer element having an element number (m, n) is represented by Equation 7.

[Equation 7]

$$\Delta\psi(m,n) = Am + Bn + D$$

$$A = \pi\left(\sin\theta\cos\phi - \sin\alpha\cos\beta\right), \qquad B = \pi\left(\sin\theta\sin\phi - \sin\alpha\sin\beta\right)$$

$$D = -\frac{N-1}{2}\left(A + B\right)$$

Equation 7 is a planar equation in an (m, n, $\Delta\Psi$) coordinate system. In the plane, $\Delta\Psi$ equals 0 ($\Delta\Psi = 0$) at x = y = 0. Therefore, by separately evaluating the change of the phase in each of the x direction and the y direction, it is possible to identify a plane represented by Equation 7.

When the delay process is executed with the reception focus point at F (r, θ, φ), because the reflection wave (received wave signal) from the point P (r, α, β) after the delay process would be shifted by the phase difference shown in Equation 7, the reflection wave is represented by Equation 8. G in Equation 8 represents a complex amplitude.

[Equation 8]

$$S(m,n) = G \cdot \exp(-j(Am + Bn))$$

Using the result of Equation 8, calculation of the two-dimensional evaluation value is reviewed. The two-dimensional evaluation value is a value indicating a degree of phasing of the received wave signals over the entirety of the plurality of transducer elements 12 that are two-dimensionally arranged, and is calculated based on the one-dimensional evaluation values in the x direction and the y direction. As the one-dimensional evaluation value, the CF (Coherence Factor), the PCF (Phase Coherence Factor), the SCF (Sign Coherence Factor), the STF (Sign Transit Factor), or the like may be used.

<CF (Coherence Factor) >

When a received wave signal at an ith transducer element 12 arranged one-dimensionally is s(i), a one-dimensional CF is calculated by Equation 9.

[Equation 9]

$$CF^{1D} = \frac{\left| \sum_{i=0}^{N-1} s(i) \right|^2}{N \cdot \sum_{i=0}^{N-1} |s(i)|^2}$$

When the received wave signal at the (m, n)th transducer element 12 arranged two-dimensionally is s(m, n), and Equation 9 is expanded to two dimensions, Equation 10 representing a two-dimensional CF may be obtained.

[Equation 10]

$$CF^{2D} = \frac{\left| \sum_{m=0}^{N-1} \sum_{n=0}^{N-1} s(m,n) \right|^2}{N^2 \cdot \sum_{m=0}^{N-1} \sum_{n=0}^{N-1} |s(m,n)|^2}$$

When Equation 8 is substituted into Equation 10, the complex amplitude G is cancelled, and, because m and n are values independent from each other, Equation 11 is obtained.

[Equation 11]

$$CF^{2D} = \frac{\left| \sum_{m=0}^{N-1} \sum_{n=0}^{N-1} \exp\left(-j(Am+Bn)\right) \right|^2}{N^2 \cdot \sum_{m=0}^{N-1} \sum_{n=0}^{N-1} \left| \exp\left(-j(Am+Bn)\right) \right|^2}$$

$$= \frac{\left| \sum_{m=0}^{N-1} \exp\left(-jAm\right) \right|^2 \cdot \left| \sum_{n=0}^{N-1} \exp\left(-jBn\right) \right|^2}{N^2 \cdot \sum_{m=0}^{N-1} \left| \exp\left(-jAm\right) \right|^2 \cdot \sum_{n=0}^{N-1} \left| \exp\left(-jBn\right) \right|^2} = CF^{1Dx} \cdot CF^{1Dy}$$

The evaluation value calculation unit 50 applies a calculation according to Equation 11 based on the plurality of the received wave signals to which the delay process is applied (refer to Equation 8) which are output from the delay processor 30, to obtain a two-dimensional evaluation value $CF^{2D}$ in the xy plane based on the one-dimensional evaluation value $CF^{1Dx}$ in the x direction and the one-dimensional evaluation value $CF^{1Dy}$ in the y direction.

<PCF (Phase Coherence Factor)>

Using a standard deviation $\sigma(\Delta\Psi(i))$ of the phases of the received wave signals in the arrangement direction of the transducer elements 12, the one-dimensional PCF is calculated by Equation 12.

[Equation 12]

$$PCF^{1D} = 1 - \frac{\gamma}{\sigma_0} \sigma\left(\Delta\psi(i)\right)$$

$$\sigma\left(\Delta\psi(i)\right) = \sqrt{\frac{1}{N} \cdot \sum_{i=0}^{N-1} \left(\Delta\psi(i)\right)^2 - \left\{\frac{1}{N} \cdot \sum_{i=0}^{N-1} \Delta\psi(i)\right\}^2} \qquad i = 0,1....N\text{-}1$$

$\sigma_0 = \pi/\,3^{1/2}$: CONSTANT FOR NORMALIZING STANDARD DEVIATION; $\gamma$: ADJUSTMENT PARAMETER

The standard deviation $\sigma(\Delta\Psi(m, n))$ when the transducer elements 12 are two-dimensionally arranged is represented by Equation 13, based on a result of Equation 7.

[Equation 13]

$$\sigma\left(\Delta\psi(m,n)\right) = \sigma\left(Am+Bn+D\right)$$

Equation 13 may be transformed into Equation 15 using a characteristic of variance shown in Equation 14.

[Equation 14]

$$\sigma^2\left(Am+Bn+D\right) = \sigma^2\left(Am\right) + \sigma^2\left(Bn\right)$$

[Equation 15]

$$\sigma\left(\Delta\psi\left(m,n\right)\right)=\sqrt{\sigma^{2}\left(Am\right)+\sigma^{2}\left(Bn\right)}$$

Therefore, when Equation 12 representing the one-dimensional PCF is expanded to two dimensions, Equation 16 representing a two-dimensional PCF is obtained. In Equation 16, in order to normalize the standard deviation expanded to two dimensions, a factor of 1/2 is introduced within the square root.

[Equation 16]

$$PCF^{2D}=1-\frac{\gamma}{\sigma_{0}}\sqrt{\frac{\sigma_{x}^{2}+\sigma_{y}^{2}}{2}} \qquad \sigma_{x}=\sigma(Am),\ \sigma_{y}=\sigma(Bn)$$

Using the one-dimensional PCF$^{1Dx}$ in the x direction and the one-dimensional PCF$^{1Dy}$ in the y direction, Equation 17 may be obtained from Equation 16.

[Equation 17]

$$PCF^{2D}=1-\frac{\gamma}{\sigma_{0}}\sqrt{\frac{\sigma_{x}^{2}+\sigma_{y}^{2}}{2}}=1-\sqrt{\frac{1}{2}\cdot\left\{\left(\frac{\gamma}{\sigma_{0}}\sigma_{x}\right)^{2}+\left(\frac{\gamma}{\sigma_{0}}\sigma_{y}\right)^{2}\right\}}=1-\sqrt{\frac{\left(1-PCF^{1Dx}\right)^{2}+\left(1-PCF^{1Dy}\right)^{2}}{2}}$$

The evaluation value calculation unit 50 applies a calculation according to Equation 17 based on the plurality of the received wave signals to which the delay process is applied and that are output from the delay processor 30, to obtain the two-dimensional evaluation value PCF$^{2D}$ in the xy plane based on the one-dimensional evaluation value PCF$^{1Dx}$ in the x direction and the one-dimensional evaluation value PCF$^{1Dy}$ in the y direction.

<SCF (Sign Coherence Factor)>

The SCF is based on a same principle as the PCF, and the received wave signal is binarized so that a standard deviation of a binarized signal b(i) is used as the index, without calculating the phase. In this case, because the standard deviation of the binarized signal b(i) has a value of 0 ~ 1, $\gamma$ and $\sigma_{0}$ in Equation 12 for the PCF are omitted, and a one-dimensional SCF is represented by Equation 18.

[Equation 18]

$$SCF^{1D}=1-\sigma\left(b(i)\right)$$

Equations 16 and 17 may be similarly calculated, so that a two-dimensional SCF is obtained as Equation 19.

[Equation 19]

$$SCF^{2D}=1-\sqrt{\frac{\sigma_{x}^{2}+\sigma_{y}^{2}}{2}}=1-\sqrt{\frac{\left(1-SCF^{1Dx}\right)^{2}+\left(1-SCF^{1Dy}\right)^{2}}{2}}$$

The evaluation value calculation unit 50 applies a calculation according to Equation 19 by binarizing the plurality of the received wave signals to which the delay process is applied and that are output from the delay processor 30, to obtain a two-dimensional evaluation value $SCF^{2D}$ in the xy plane based on the one-dimensional evaluation value $SCF^{1Dx}$ in the x direction and the one-dimensional evaluation value $SCF^{1Dy}$ in the y direction. Alternatively, the evaluation value calculation unit 50 may obtain the two-dimensional evaluation value by obtaining a p-th power of $SCF^{2D}$ using an adjustment coefficient p.

<STF (Sign Transit Factor)>

A one-dimensional STF is calculated from Equation 20 from a zero-cross density (which corresponds to an average frequency) of the received wave signals in the arrangement direction of the transducer element 12.

[Equation 20]

$$STF^{1D} = \frac{1}{N-1}\sum_{i=0}^{N-2} c(i)$$

$$c(i) = \left\{ \begin{array}{ll} 1 & \text{if } \text{sign}(s(i)) \neq \text{sign}(s(i+1)) \\ 0 & \text{if } \text{sign}(s(i)) = \text{sign}(s(i+1)) \end{array} \right.$$

In the case of the two-dimensional array transducer 10, as shown in Equation 7, a phase difference between a reflection wave (received wave signal) from the reception focus point F (r, θ, φ) and the reflection wave (received wave signal) from the point P (r, α, β) at the transducer element 12 having an element number of (m, n) is a planar equation. In addition, the received wave signal from the point P (r, α, β) after the delay process is as shown in Equation 8.

FIG. 4 is a diagram showing a wave plane of the received wave signal to which the delay process is applied. In FIG. 4, the xy coordinate system corresponds to the transducer plane of the two-dimensional array transducer 10, and solid lines in the xy coordinate system show portions where the phase is 2nn [rad] (where n is an integer). In addition, broken lines in the xy coordinate system show portions where the phase is (2n + 1)π [rad] (where n is an integer).

As an index indicating the degree of phasing, in the wave plane shown in FIG. 4, an average frequency $f_{2D}$ in the direction of the maximum frequency is used. The average frequency $f_{2D}$ can be calculated by Equation 21 based on a frequency $f_x$ in the x direction and a frequency fy in the y direction.

[Equation 21]

$$f_{2D} = \frac{f_x^2 + f_y^2}{f_x \cdot f_y}$$

As the zero-cross density corresponds to the average frequency, if the STF is expanded to two dimensions based on Equation 21, a two-dimensional STF is obtained as Equation 22.

[Equation 22]

$$STF^{2D} = \frac{(STF^{1Dx})^2 + (STF^{1Dy})^2}{STF^{1Dx} \cdot STF^{1Dy}}$$

The evaluation value calculation unit 50 applies a calculation according to Equation 22 based on the plurality of the received wave signals to which the delay process is applied and that are output from the delay processor 30, to obtain the two-dimensional evaluation value $STF^{2D}$ in the xy plane based on the one-dimensional evaluation value $STF^{1Dx}$ in the x direction and the one-dimensional evaluation value $STF^{1Dy}$ in the y direction. As a higher zero-cross density indicates a signal at a further distance from the main lobe (that is, the signal is a signal which should be reduced), the two-dimensional evaluation value may be obtained, for example, by obtaining a p-th power of (1 - $STF^{2D}$) using an adjustment coefficient p.

The calculation of the two-dimensional evaluation value using the CF, the PCF, the SCF, and the STF is as follows. Because the phase difference shown in Equation 7 is a planar equation, when the one-dimensional evaluation value is calculated in the x direction, for example, theoretically, the same result is obtained in any line along the x direction. Therefore, for example, one line arranged along the x direction in FIG. 2 may be set as a representative line, and the one-dimensional evaluation value in the x direction may be calculated based on the plurality of the received wave signals to which the delay process is applied, obtained from the plurality of transducer elements 12 included in the representative line. As the representative line, for example, a line passing through a center or near the center of the transducer plane is preferable. Similarly, when the one-dimensional evaluation value is to be calculated for the y direction, one line arranged along the y direction is set as a representative line. Alternatively, a plurality of lines may be set as representative lines, and an average for the plurality of lines may be set as the one-dimensional evaluation value.

Because the number of elements of the transducer elements 12 in the two-dimensional array transducer 10 is large, in some cases, channel reduction is performed in the probe. In this case, the evaluation value calculation unit 50 may calculate the one-dimensional evaluation value based on the received wave signals after the channel reduction, and may obtain the two-dimensional evaluation value based on the one-dimensional evaluation values.

A preferred embodiment of the present invention has been described. The above-described embodiment, however, is merely exemplary in every aspect, and in no way limits the scope of the present invention. The present invention includes various modified configurations within the scope and spirit of the invention.

[Explanation of Reference Numerals]

10 TWO-DIMENSIONAL ARRAY TRANSDUCER; 12 TRANSDUCER ELEMENT; 20 TRANSMITTING UNIT; 30 DELAY PROCESSOR; 32 DELAY CIRCUIT; 40 SUMMATION PROCESSOR; 50 EVALUATION VALUE CALCULATION UNIT; 60 MULTIPLICATION UNIT; 70 IMAGE FORMATION UNIT; 72 DISPLAY; 80 CONTROLLER.

**Claims**

1.  An ultrasound diagnostic apparatus, comprising:

    a plurality of transducer elements that are two-dimensionally arranged;
    a delay processor that applies a delay process on a plurality of received wave signals obtained from the plurality of transducer elements, to phase the received wave signals;
    an evaluation value calculation unit that evaluates a degree of phasing based on the plurality of received wave signals to which the delay process is applied, to obtain a two-dimensional evaluation value related to the plurality of transducer elements that are two-dimensionally arranged;
    a summation processor that applies a summation process to the plurality of received wave signals to which the delay process is applied, to obtain a reception signal; and
    a signal adjusting unit that adjusts the reception signal based on the two-dimensional evaluation value, to reduce an unnecessary signal component.

2.  The ultrasound diagnostic apparatus according to Claim 1, wherein
    the evaluation value calculation unit calculates a plurality of one-dimensional evaluation values based on the plurality of received wave signals to which the delay process is applied, and calculates the two-dimensional evaluation value based on the plurality of the one-dimensional evaluation values.

3.  The ultrasound diagnostic apparatus according to Claim 1, wherein
    the evaluation value calculation unit evaluates, for the plurality of transducer elements that are two-dimensionally arranged, a degree of phasing for each arrangement direction for a plurality of arrangement directions that differ from each other, to calculate a one-dimensional evaluation value, and calculates the two-dimensional evaluation value indicating a degree of phasing over the entirety of the two-dimensional arrangement based on a plurality of the one-dimensional evaluation values obtained from the plurality of arrangement directions.

4. The ultrasound diagnostic apparatus according to Claim 3, wherein
the evaluation value calculation unit calculates, for each arrangement direction, the one-dimensional evaluation value for the arrangement direction based on a plurality of received wave signals obtained from transducer elements of at least one line along the arrangement direction.

5. The ultrasound diagnostic apparatus according to Claim 3, wherein
the evaluation value calculation unit calculates the one-dimensional evaluation value for each arrangement direction for two arraignment directions that differ from each other, and calculates the two-dimensional evaluation value based on two one-dimensional evaluation values obtained from the two arrangement directions.

6. The ultrasound diagnostic apparatus according to Claim 5, wherein
the evaluation value calculation unit calculates, for each arrangement direction, the one-dimensional evaluation value for the arrangement direction based on a plurality of received wave signals obtained from transducer elements of at least one line along the arrangement direction.

7. The ultrasound diagnostic apparatus according to Claim 3, wherein
the evaluation value calculation unit calculates the one-dimensional evaluation value for each arrangement direction for two arrangement directions that are orthogonal to each other, and calculates the two-dimensional evaluation value based on two one-dimensional evaluation values obtained from the two arrangement directions.

8. The ultrasound diagnostic apparatus according to Claim 7, wherein
the evaluation value calculation unit calculates, for each arrangement direction, the one-dimensional evaluation value for the arrangement direction based on a plurality of received wave signals obtained from transducer elements of at least one line along the arrangement direction.

9. The ultrasound diagnostic apparatus according to Claim 3, wherein
the evaluation value calculation unit calculates, as the one-dimensional evaluation value, a CF (Coherence Factor), a PCF (Phase Coherence Factor), an SCF (Sign Coherence Factor), or an STF (Sign Transit Factor).

10. The ultrasound diagnostic apparatus according to Claim 5, wherein
the evaluation value calculation unit calculates, as the one-dimensional evaluation value, a CF (Coherence Factor), a PCF (Phase Coherence Factor), an SCF (Sign Coherence Factor), or an STF (Sign Transit Factor).

11. The ultrasound diagnostic apparatus according to Claim 7, wherein
the evaluation value calculation unit calculates, as the one-dimensional evaluation value, a CF (Coherence Factor), a PCF (Phase Coherence Factor), an SCF (Sign Coherence Factor), or an STF (Sign Transit Factor).

12. The ultrasound diagnostic apparatus according to Claim 1, wherein
the signal adjusting unit multiplies the reception signal to which the summation process is applied, obtained from the summation processor, and the two-dimensional evaluation value obtained from the evaluation value calculation unit, to adjust the reception signal to which the summation process is applied, and to reduce the unnecessary signal component.

FIG. 1

10:TWO-DIMENSIONAL ARRAY TRANSDUCER

12:TRANSDUCER ELEMENT

FIG. 2

10:TWO-DIMENSIONAL
ARRAY TRANSDUCER

12: TRANSDUCER
ELEMENT (x, y)

y

$\varphi$

x

$\theta$

P(r,α,β)

F(r,θ,φ)

z

# FIG. 3

y

1/f$_{2D}$

1/f$_y$

x

1/f$_x$

# FIG. 4

## INTERNATIONAL SEARCH REPORT

| International application No. |
| --- |
| PCT/JP2013/084685 |

**A. CLASSIFICATION OF SUBJECT MATTER**
*A61B8/00(2006.01)i*

According to International Patent Classification (IPC) or to both national classification and IPC

**B. FIELDS SEARCHED**

Minimum documentation searched (classification system followed by classification symbols)
A61B8/00

Documentation searched other than minimum documentation to the extent that such documents are included in the fields searched
Jitsuyo Shinan Koho       1922-1996   Jitsuyo Shinan Toroku Koho   1996-2014
Kokai Jitsuyo Shinan Koho   1971-2014   Toroku Jitsuyo Shinan Koho   1994-2014

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y<br>A | JP 2012-231819 A  (Konica Minolta Medical & Graphic, Inc.),<br>29 November 2012 (29.11.2012),<br>entire text; all drawings<br>& US 2012/0277589 A1 | 1-2,12<br>3-11 |
| Y<br>A | JP 2012-152311 A  (Hitachi Aloka Medical, Ltd.),<br>16 August 2012 (16.08.2012),<br>entire text; all drawings<br>& US 2012/0190985 A1    & EP 2479587 A1<br>& CN 102599929 A | 1-2,12<br>3-11 |
| Y<br>A | JP 7-65146 A  (Toshiba Corp.),<br>10 March 1995 (10.03.1995),<br>paragraph [0039]; fig. 9<br>(Family: none) | 1-2,12<br>3-11 |

☐ Further documents are listed in the continuation of Box C.      ☐ See patent family annex.

| | |
| --- | --- |
| *     Special categories of cited documents:<br>"A"   document defining the general state of the art which is not considered    to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>    21 February, 2014 (21.02.14) | Date of mailing of the international search report<br>    11 March, 2014 (11.03.14) |
| --- | --- |
| Name and mailing address of the ISA/<br>    Japanese Patent Office | Authorized officer |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US P5910115 A

- JP 2012081114 A

**Non-patent literature cited in the description**

- **J. CAMECHO et al.** Phase Coherence Imaging. *IEEE trans. UFFC,* 2009, vol. 56 (5

- **PAI-CHI LI et al.** Adaptive Imaging Using the Generalized Coherence Factor. *IEEE trans. UFFC,* 2003, vol. 50 (2